# FASCICULE DE BREVET EUROPEEN

(11) **EP 0 950 232 B1**
(45) Date de publication et mention de la délivrance du brevet: **26.02.2003**
(21) Numéro de dépôt: 97953983.0
(22) Date de dépôt: 31.12.1997
(51) Int. Cl.: G08B 25/01

(54) **SYSTEME DE SURVEILLANCE ET D'ASSISTANCE POUR PERSONNES ISOLEES ET DISPOSITIF POUR LA MISE EN OEUVRE DE CE SYSTEME**
SYSTEM UND VORRICHTUNG ZUM ÜBERWACHEN UND UNTERSTÜTZEN VON ISOLIERTEN PERSONEN
SYSTEM FOR MONITORING AND ASSISTING ISOLATED PERSONS AND DEVICE FOR IMPLEMENTING THE SYSTEM

(30) Priorité: 31.12.1996 FR 9616386
(43) Date de publication de la demande: 20.10.1999
(73) Titulaire: Cadet, Pierre-Henri, 06400 Cannes (FR); Vigneron, Marc, 69004 Lyon (FR); Poyeton, Laurent, 69006 Lyon (FR)
(72) Inventeur: Cadet, Pierre-Henri, 06400 Cannes (FR); Vigneron, Marc, 69004 Lyon (FR); Poyeton, Laurent, 69006 Lyon (FR)
(74) Mandataire: Vuillermoz, Bruno
(86) Numéro de dépôt international: FR9702466
(87) Numéro de publication internationale: WO98029852

(56) Documents cités:
- DE-A- 4 325 087
- DE-U- 9 408 119
- FR-A- 2 598 535

## Description

L'invention concerne un système destiné à permettre la surveillance et l'assistance de personnes isolées, notamment en environnement domestique, c'est à dire à domicile, mais également professionnel, industriel, et à l'occasion d'activités de loisirs. Elle concerne également un dispositif permettant la mise en oeuvre de ce système.

Dans le cadre de l'environnement domestique, les récentes études démographiques effectuées parmi les populations des pays industrialisés montrent un accroissement significatif du nombre des personnes âgées, et corollairement des personnes infirmes, malades ou handicapées, beaucoup de ces personnes vivant seules.

Par ailleurs, si pendant longtemps, l'hospitalisation de ces personnes au moindre incident était la règle, la tendance actuelle, tant dans le but d'optimiser le confort des patients, que dans celui de la réduction des coûts d'hospitalisation, vise à promouvoir autant que faire se peut, le maintien de ces personnes chez elles, en proposant soit un service médicalisé à domicile, soit un service de surveillance.

Le problème posé par le maintien à leur domicile de ces personnes seules, réside dans les risques de malaise, chutes, et autres problèmes médicaux susceptibles d'affecter ces personnes et, leurs pendants en termes de conséquence si elles restent dépourvues de tout soin pendant une période plus ou moins longue.

Bien que des visites périodiques de familiers, d'infirmières et autres, confèrent une certaine sécurité, et quand bien même la présence du téléphone permet en cas de besoin d'alerter les services compétents, certaines situations demeurent sans solution, notamment en cas de malaise, ou de chute, interdisant à la personne concernée l'utilisation du téléphone, et partant, la possibilité d'avoir recours à des soins.

Afin de résoudre cette difficulté, il a été proposé, des systèmes de surveillance, d'assistance et/ou de garde, dans lesquels la personne seule porte en permanence sur elle, un boîtier, qui, lorsqu'il est sollicité par ladite personne, induit l'émission d'un signal en direction d'un dispositif centralisé, propre à son tour à induire le déclenchement d'une alerte ou d'une alarme au niveau d'un service de surveillance, des services de secours et/ou des proches, et ce, par le biais du réseau téléphonique commuté. De tels systèmes ont par exemple été décrits dans les documents EP-A-0 522 660 ou US-A-3 914 692, ou encore FR-A-2 598 535.

Indiscutablement, le système ainsi proposé constitue un progrès certain. Cependant, il n'a pu prendre en compte toutes les situations auxquelles sont confrontées les personnes seules, notamment lorsque celles-ci chutent, induisant une perte de connaissance, ou un état tel qu'elles ne sont plus en mesure d'actionner le boîtier qu'elles portent.

Les mêmes problèmes se posent, notamment dans le domaine industriel, et plus particulièrement en atmosphère hostile, où l'inhalation de produits toxiques, ou l'exposition à des risques potentiels, peuvent entraîner la chute directe, ou consécutive à un malaise, et dont le maintien dans une telle atmosphère peut s'avérer rédhibitoire.

Afin de s'affranchir de cet inconvénient, on a proposé dans le document DE-A-4 325 un système intégrant un détecteur de choc, et partant, de chute. Malheureusement, l'organe de détection de choc mis en oeuvre ne permet pas de s'affranchir des fausses alertes, inhérentes à des mouvements brusques, par exemple.

L'objet de l'invention est de proposer un système de surveillance et d'assistance pour personnes isolées, tenant compte de ces risques.

Ce système comprend :
- une unité délocalisée au niveau du lieu de surveillance et d'assistance desdites personnes, munie d'un récepteur et destinée à transférer un signal d'alerte, par exemple par le biais du réseau téléphonique commuté, en un ou plusieurs lieux déterminés, notamment centre de surveillance, centre de secours, proches, famille ;
- et une unité portable, destinée à être portée par lesdites personnes, et intégrant un émetteur destiné à émettre un ou des signaux en direction de l'unité délocalisée, lesdits signaux étant destinés à induire le transfert du signal d'alerte par celle-ci .

Il se caractérise en ce que l'unité portable intègre en outre des moyens de détection de la chute de la personne qui la porte, susceptibles d'induire l'émission par l'émetteur qu'elle intègre desdits signaux destinés à leur tour à induire le transfert d'alerte, ces moyens de détection de chutes étant constitués d'un moyen gravimétrique, tel que notamment un détecteur à bille de mercure, ou accélérométrique, tel qu'un accéléromètre piezo-électrique.

On s'affranchit par ce biais du risque du maintien en situation difficile voire critique, de personnes dont les études épidémiologiques récentes montrent que le nombre est relativement important, et que les conséquences en termes de traitement sont également critiques.

Dans le cadre de la mise en oeuvre de moyens de détection accélérométriques, on munit avantageusement l'unité portable de trois accéléromètres, situés selon trois directions différentes, de telle sorte à détecter les chutes dans le plan vertical, les chutes dans le plan latéral, et les chutes d'avant en arrière.

Selon une autre caractéristique de l'invention, l'unité portable est également munie d'un organe actionnable par la personne qui le porte, destiné à induire également, et ce de manière connue, le transfert d'alerte par l'unité délocalisée. Cet organe est le plus souvent constitué d'un bouton poussoir, mais peut bien entendu être constitué par tout autre type d'organes remplissant une fonction équivalente. Cet organe peut par exemple être intégré au niveau d'un collier de suspension de l'unité portable, qui en cas d'arrachement, induit le transfert d'alerte.

Selon une autre caractéristique de l'invention, l'unité portable est munie d'un interrupteur d'impédance cutanée. De la sorte, le système est en état de veille que lorsque l'unité portable est effectivement portée par l'utilisateur, la peau fermant le circuit de veille intégrée au sein de ladite unité portable.

Selon une autre caractéristique de l'invention, l'unité portable intègre un circuit de recueil et de stockage de l'électrocardiogramme de la personne considérée. A cet effet, l'unité portable est pourvue d'un collier venant se positionner autour du cou de la personne concernée, ledit collier étant muni au niveau de son extrémité, c'est à dire au niveau opposé à la zone de fixation de l'unité portable, et donc, lorsqu'il est en place derrière le cou, d'électrodes de contact, susceptibles de prendre une première information dans le cadre du recueil de l'électrocardiogramme. De plus, le boîtier constitutif de l'unité portable comporte lui-même une électrode, située sur sa face dorsale, afin d'être situé au niveau du manubrium sternal de l'individu, de telle sorte à permettre en liaison avec l'électrode du cou, l'acquisition des données nécessaires à la réalisation d'un électrocardiogramme, celui-ci étant stocké dans une mémoire intégrée au sein dudit boîtier. De la sorte, il devient possible en cas de malaise, ou en cas de détection de chute, de déceler très rapidement la cause du malaise ou de la chute, et le cas échéant, apporter les soins appropriés dans les meilleurs délais.

La manière dont l'invention peut être réalisée et les avantages qui en découlent ressortiront mieux de l'exemple de réalisation qui suit donné à titre indicatif et non limitatif à l'appui des figures annexées.

La figure 1 est une représentation schématique sous la forme d'un synoptique de l'unité portable conforme à l'invention.

La figure 2 est une représentation schématique du fonctionnement du système conforme à l'invention..

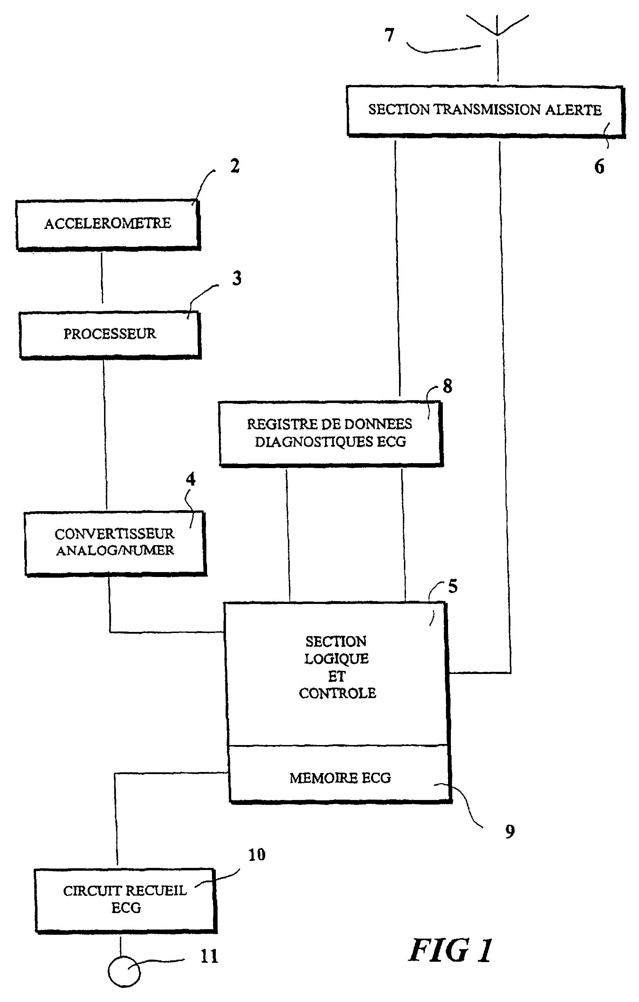

La figure 1 représente donc un schéma synoptique d'une forme de réalisation préférée de l'unité portable de l'invention. Cette unité portable est destinée à être portée par la personne, généralement une personne âgée ou un travailleur en atmosphère hostile.

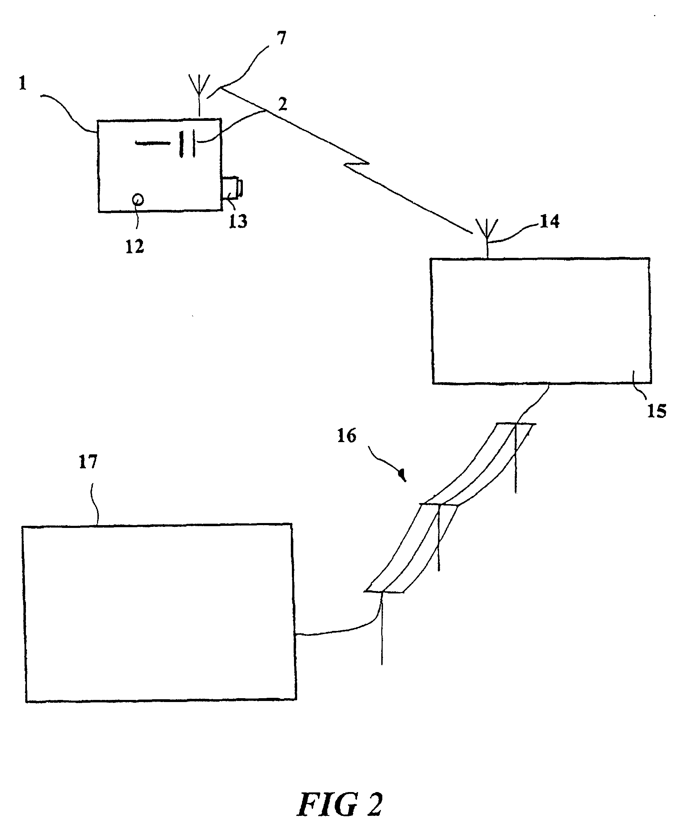

Cette unité portable se présente traditionnellement sous la forme d'un boîtier (1) de faibles dimensions, par exemple de 40 x 50 mm, et de masse réduite, intégrant un certain nombre de composants électroniques et tout d'abord, trois accéléromètres (2), ainsi qu'on peut l'observer sur la figure 2. Ces accéléromètres sont calibrés pour détecter une chute selon trois plans, à savoir le plan vertical, le plan latéral et le plan antéro-postérieur. Ces accéléromètres sont de plus calibrés, de telle sorte à émettre un signal relatif à une chute, pour tout mouvement subissant une accélération d'environ 2g pour une masse d'environ 50 kilos et d'une hauteur d'environ un mètre.

L'objet de l'invention n'est pas de décrire un tel accéléromètre bien connu en soi. De manière classique, il est constitué d'un cristal piezo électrique, dont la déformation entraîne la création d'une tension électrique, interprétée ensuite par un circuit électronique, notamment au niveau d'un processeur (3), puis convertie en un signal numérique au moyen d'un convertisseur analogique/numérique (4), avant d'être transféré au niveau d'une section logique et de contrôle (5).

Ces accéléromètres peuvent également être du type électronique au silicium.

La section logique et de contrôle comporte typiquement un logiciel apte, en fonction du signal transmis par le ou les accéléromètres (2), à induire l'émission d'un signal par le biais d'une section de transmission d'alerte (6) et une antenne (7) en direction d'une unité délocalisée (14), typiquement un démodulateur, mise en place au niveau de l'habitation de la personne considérée et à portée de ladite unité portable. On conçoit de fait qu'il est possible de paramétrer le seuil d'émission du signal d'alerte en jouant au niveau du logiciel de traitement du signal transmis par les accéléromètres, et partant, de conférer une certaine fiabilité à l'ensemble en s'affranchissant des fausses alertes.

Cette unité délocalisée est en soi connue, et est destinée après réception d'un signal d'alerte émis par l'unité portable, à induire à son tour le transfert ou la transmission d'un signal d'alerte, par exemple par le biais du réseau téléphonique commuté (16), au niveau d'un service de surveillance, d'un service de secours et/ou de proches (17), afin d'indiquer l'occurrence d'un risque chez la personne considérée.

Le ou les accéléromètres utilisés, ainsi que le réseau électronique de transmission du signal, sont réalisés de telle sorte à réduire au minimum les risques d'interférence, notamment de mouvements brutaux, de telle sorte à fiabiliser le plus possible la détection d'un incident ou d'un accident réel.

L'unité portable (1) est alimentée électriquement au moyen de piles ou batteries traditionnelles (non représentées). De même, l'unité délocalisée (14) est alimentée électriquement par le secteur domestique. Il peut être prévu une plusieurs batteries, susceptible de maintenir ladite unité délocalisée en état de fonctionnement pendant quelques heures, en cas de coupure de courant ou de panne d'électricité.

Par ailleurs, l'entité constituée par l'unité portable (1) et l'unité délocalisée (15) est parfaitement référencée, afin d'être identifiable lors du transfert d'alerte par le centre de secours ou de surveillance (17). A cet effet, une codification binaire propre à chacune de ces entités est introduite dans la mémoire de la section logique et de contrôle (5), cette codification étant véhiculée par la porteuse du réseau téléphonique en cas d'alerte.

Selon une variante de l'invention, l'unité portable (1) est miniaturisée, de telle sorte à se présenter sous la forme d'un microcircuit contenu dans un adhésif, et mieux connu sous l'expression en langue anglaise « patch ».

Selon une autre variante, cette unité portable est intégrée au sein d'une montre.

Dans tous ces cas, et afin de s'assurer que l'unité portable est effectivement portée par l'utilisateur auquel elle est destinée, celle-ci intègre un circuit électrique muni d'un interrupteur d'impédance cutanée. En d'autres termes, on utilise la conduction électrique de la peau pour fermer ledit circuit électrique, qui émet alors un signal, notamment en direction de l'unité centrale, indiquant que l'unité portable est opérationnelle et dûment portée.

Dans une variante de la forme de réalisation précédente, on munit en outre ledit circuit électrique d'un système de temporisation, typiquement d'une durée égale à environ 30 secondes, de telle sorte à ce qu'en l'absence de contact avec la peau de l'utilisateur, et par conséquent en cas d'ouverture dudit circuit, il ne se déclenche pas de signal d'alerte indiquant que l'unité portable n'est plus portée. Ceci est notamment intéressant, lorsque l'unité portable est suspendue à un collier, et ne se trouve plus en contact avec la peau lorsque l'utilisateur se penche.

Selon une forme de réalisation préférée de l'invention, l'unité portable (1) comporte également des moyens propres à permettre l'enregistrement d'un électrocardiogramme du patient. Cet enregistrement est rendu possible d'une part, par l'association au boîtier portable constitutif de l'unité portable, d'un collier (non représenté), intégrant à son extrémité supérieure une ou deux électrodes de détection, dont le simple contact avec le cou permet la prise de mesure, et d'autre part, par la mise en place au dos du boîtier (1) d'une autre électrode de mesure. A cet effet, la longueur du collier est réglée de telle sorte que, l'électrode du dos du boîtier vienne au contact du patient sensiblement au niveau du manubrium stemal. On définit de la sorte des moyens d'acquisition (11) des données nécessaires à l'établissement d'un électrocardiogramme (= ECG) associés à un circuit de recueil (10) de l'ECG. Ce circuit de recueil de l'ECG (10) est également mis en place au sein du boîtier constitutif de l'unité portable (1).

Cet électrocardiogramme est stocké en permanence dans une mémoire (9) associée à la section logique et de contrôle (5), par écrasement de la mémoire préalablement stockée. Typiquement, la durée mémorisée est de 15 mn.

La section logique et de contrôle (5) est également munie d'un sélecteur propre à enregistrer dans un registre de diagnostic (8), en d'autres termes dans une mémoire, ledit électrocardiogramme, de telle sorte à permettre au praticien d'analyser l'électrocardiogramme, et partant de détecter l'origine de la cause du malaise ou de la chute, et partant d'apporter les soins adéquats, de manière plus rapide.

Avantageusement, l'électrocardiogramme est analysé automatiquement en temps réel au sein de la section logique et de contrôle, afin de déceler une bradycardie ou une tachycardie majeure, en paramétrant un seuil à ne pas dépasser, respectivement de basses fréquences, par exemple 25 pulsations par minute, ou de hautes fréquences, par exemple 150 pulsations par minute.

Il devient alors possible de générer le déclenchement d'un signal d'alerte en cas de malaise ou de perte de connaissance, phénomènes induisant une variation mesurable du rythme cardiaque.

En outre, selon une version évoluée de l'invention, la mémoire de la section logique et de contrôle (5) est chargée avec la gestion de la posologie d'un ou de plusieurs médicaments appropriés à la maladie dont souffre éventuellement chroniquement le porteur de l'unité portable. Le contenu de cette mémoire peut être accessible par le biais d'un lecteur muni d'une connexion appropriée, permettant le cas échéant aux premiers secours, d'avoir accès aux renseignements essentiels du traitement à mettre en oeuvre.

Selon l'invention, le boîtier comporte également un bouton poussoir (13) ou analogue, susceptible d'être actionné par la personne qui porte l'unité portable (1) ou par un tiers, afin de déclencher une alarme en cas de nécessité. Ce bouton poussoir peut être remplacé ou doublé par un organe électrique (non représenté), par exemple intégré dans le dispositif de fermeture dudit collier, et fermant le circuit électrique permettant le déclenchement du signal d'alerte, cet organe électrique étant activé par l'arrachement du collier supportant l'unité portable. De fait, compte tenu de cette possibilité, le déclenchement du signal d'alerte peut s'avérer plus facile lorsque l'utilisateur est en situation difficile, rendant quelque fois délicat l'actionnement d'un bouton poussoir traditionnel. En outre, il permet de s'affranchir des fausses alertes consécutives à l'actionnement par inadvertance du bouton-poussoir.

Avantageusement, et dans le but de sécuriser le plus possible la personne isolée, le système de surveillance et/ou d'assistance conforme à l'invention comporte des moyens permettant d'informer ladite personne que le signal d'alerte a bien été transmis au centre de surveillance, de secours ou auprès des tiers désignés. A cet effet, un signal transite par le biais du réseau téléphonique commuté à partir dudit centre ou du ou des tiers vers l'unité délocalisée (15) bien entendu après avoir effectivement transmis l'alerte auprès du service concerné, ce signal étant matérialisé par exemple par l'éclairement du diode électro-luminescente (12) sur l'unité portable (1), par la vibration d'un petit buzzer (non représenté), voire par l'émission d'un message sonore pré-enregistré, de telle manière que la personne en détresse puisse visualiser ou entendre la confirmation du bon déclenchement du signal d'alerte.

Par ailleurs, afin de s'assurer du bon fonctionnement du système, et notamment de l'entité unité portable (1)/unité délocalisée (15), on munit avantageusement ces deux unités d'un testeur (non représenté), constitué par exemple d'un bouton-poussoir non directement accessible, afin de ne pas risquer de fausses manoeuvres, dont l'activation engendre un auto-test des fonctions essentielles du système sans engendrer de transfert d'alerte. Le bon fonctionnement de ladite entité peut alors se matérialiser, après enclenchement du testeur, par l'éclairement d'une diode électro-luminescente d'une couleur différente de celle (12) matérialisant la bonne transmission du signal d'alerte.

Bien entendu, l'unité délocalisée peut être montée au niveau du fauteuil roulant d'une personne handicapée. Dans cette configuration, le système conforme à l'invention remplit parfaitement son rôle, l'interface avec le réseau commuté étant à ce jour parfaitement maîtrisé, notamment en reprenant les développements mis en oeuvre pour les téléphones mobiles cellulaires.

Dans le même ordre d'idée, il est également possible de mettre en oeuvre un interface de localisation intégré au sein de l'unité portable et de l'unité délocalisée, pour par exemple permettre le repérage et le positionnement par satellite - type GPS, en munissant le satellite d'un boîtier interface complémentaire. Cette forme de réalisation trouve application lorsque l'utilisateur est intellectuellement déficient, et qu'il convient de retrouver sa trace.

De la sorte, on dispose d'un ensemble complet, susceptible de sécuriser outre les personnes seules, notamment dans un environnement domestique, également leur entourage, dans la mesure où dans presque toutes les situations possibles, de détresse notamment, une alerte peut être transmise aux services ou aux personnes concernées. Par ailleurs, un tel système est largement applicables aux personnes travaillant isolément en atmosphère hostile.

On conçoit donc tout l'intérêt d'un tel système et de l'unité portable associée.

## Revendications

1. Système de surveillance et d'assistance pour personne(s) isolée(s), comprenant :
- une unité (15) délocalisée au niveau du lieu souhaité de surveillance ou d'assistance desdites personnes, cette unité étant munie d'un récepteur et étant destinée à transférer un signal d'alerte, notamment par le biais du réseau téléphonique commuté (16), en un ou plusieurs lieux déterminés (17), notamment centre de surveillance, centre de secours, proches, famille ;
- et une unité portable (1), destinée à être portée par lesdites personnes, et intégrant un émetteur destiné à émettre un ou des signaux en direction de l'unité délocalisée (15), lesdits signaux étant destinés à induire le transfert du signal d'alerte par le biais de ladite unité délocalisée en direction desdits lieux déterminés (17) ;
***caractérisé* en ce que** l'unité portable (1) intègre en outre des moyens de détection (2, 3, 4) de la chute de la personne qui la porte, susceptibles d'induire l'émission par l'émetteur qu'elle intègre desdits signaux destinés à leur tour à induire le transfert d'alerte par l'unité délocalisée (15), ces moyens de détection étant constitués de moyens accélérométriques constitués d'un ou plusieurs accéléromètres piezo-électriques (2).

2. Système de surveillance et d'assistance pour personnes isolées selon la revendication 1, ***caractérisé* en ce que** l'unité portable comporte trois accéléromètres (2), situés selon trois directions différentes, de telle sorte à détecter les chutes dans le plan vertical, les chutes dans le plan latéral, et les chutes d'avant en arrière.

3. Système de surveillance et d'assistance pour personnes isolées selon l'une des revendications 1 à 3, ***caractérisé* en ce que** l'unité portable (1) est également munie d'un organe actionnable (13) par la personne qui le porte, destiné à induire le transfert d'alerte par l'unité délocalisée (15).

4. Système de surveillance et d'assistance pour personnes isolées selon la revendication *3,* ***caractérisé* en ce que** l'organe actionnable (13) est constitué d'un bouton poussoir.

5. Système de surveillance et d'assistance pour personnes isolées selon la revendication 3, ***caractérisé* en ce que** l'unité portable est suspendue à un collier, et **en ce que** l'organe actionnable (13) est intégré dans le dispositif de fermeture dudit collier, l'arrachement dudit collier entraînant la genèse du signal d'alerte.

6. Système de surveillance et d'assistance pour personnes isolées selon l'une des revendication 1 à 5, ***caractérisé* en ce que** l'unité portable est fixée par adhésif sur la peau de la personne à surveiller et à assister, à l'instar d'un patch.

7. Système de surveillance et d'assistance pour personnes isolées selon l'une des revendications 1 à 6, ***caractérisé* en ce que** l'unité portable comporte un circuit électrique de veille fermé par un interrupteur d'impédance cutanée, de sorte que ladite unité portable est en état de veille que lorsqu'elle est portée.

8. Système de surveillance et d'assistance pour personnes isolées selon l'une des revendications 1 à 7, ***caractérisé* en ce que** l'unité portable (1) intègre un circuit de recueil (10, 11) et de stockage (8, 9) de l'électrocardiogramme de la personne considérée.

9. Système de surveillance et d'assistance pour personnes isolées selon la revendication 8, ***caractérisé* en ce que** l'unité portable (1) est pourvue d'un collier venant se positionner autour du cou de la personne concernée, ledit collier étant muni au niveau de son extrémité, c'est à dire au niveau opposé à la zone de fixation de l'unité portable, et donc, lorsqu'il est en place derrière le cou, d'électrodes de contact, susceptibles de prendre une première information dans le cadre du recueil de l'électrocardiogramme, et **en ce que** le boîtier constitutif de l'unité portable comporte lui-même une électrode, située sur sa face dorsale, afin d'être situé au niveau du manubrium sternal de l'individu, de telle sorte à permettre en liaison avec l'électrode du cou, l'acquisition des données nécessaires à la réalisation d'un électrocardiogramme, celui-ci étant stocké dans une mémoire intégrée au sein dudit boîtier.

10. Système de surveillance et d'assistance pour personnes isolées selon la revendication 9, ***caractérisé* en ce que** l'unité portable intègre une section logique et de contrôle apte notamment à analyser automatiquement et en temps réel l'électrocardiogramme acquis afin de déceler un dysfonctionnement cardiaque et induire alors la genèse d'un signal d'alerte.

11. Système de surveillance et d'assistance pour personnes isolées selon l'une des revendications 1 à 10, ***caractérisé* en ce que** l'unité délocalisée est montée au niveau du fauteuil roulant d'une personne handicapée, l'interface avec le réseau commuté étant du type de ceux mis en oeuvre pour le fonctionnement des téléphones portables.

12. Système de surveillance et d'assistance pour personnes isolées selon l'une des revendications 1 à 11, ***caractérisé* en ce qu'**il intègre un dispositif de repérage par satellite.

13. Système de surveillance et d'assistance pour personnes isolées selon l'une des revendications 1 à 12, ***caractérisé* en ce qu'**il comporte des moyens (12) permettant d'informer ladite ou lesdites personnes que le signal d'alerte a bien été transmis au centre de surveillance, de secours ou auprès des tiers désignés.

14. Système de surveillance et d'assistance pour personnes isolées, constitué d'une unité portable (1) intégrant un émetteur, destiné à émettre un ou des signaux susceptibles d'induire le transfert d'un signal d'alerte en direction d'un centre de surveillance, de secours ou de tiers, ***caractérisé* en ce qu'**il intègre en outre des moyens de détection (2, 3, 4) de la chute de la personne qui la porte, susceptibles d'induire l'émission par l'émetteur qu'il intègre desdits signaux destinés à induire le transfert d'alerte, ces moyens de détection étant constitués de moyens accélérométriques constitués d'un ou plusieurs accéléromètres piezo-électriques (2).

15. Système de surveillance et d'assistance pour personnes isolées selon la revendication 14, ***caractérisé* en ce qu'**il est muni d'un collier venant se positionner autour du cou de la personne concernée, ledit collier étant muni au niveau de son extrémité, c'est à dire au niveau opposé à la zone de fixation de l'unité portable, et donc, lorsqu'il est en place derrière le cou, d'électrodes de contact, susceptibles de prendre une première information dans le cadre du recueil de l'électrocardiogramme, et **en ce qu'**il comporte lui-même une électrode, située sur sa face dorsale, afin d'être situé au niveau du manubrium sternal de l'individu, de telle sorte à permettre en liaison avec l'électrode du cou, l'acquisition des données nécessaires à la réalisation d'un électrocardiogramme, celui-ci étant stocké dans une mémoire intégrée au sein dudit boîtier.

16. Système de surveillance et d'assistance pour personnes isolées selon l'une des revendications 14 et 15, ***caractérisé* en ce que** l'unité portable comporte un circuit électrique de veille fermé par un interrupteur d'impédance cutanée, de sorte que ladite unité portable est en état de veille que lorsqu'elle est portée.

## Claims

1. System for monitoring and assisting (an) isolated person(s), comprising :
- a unit (15) decentralized at the desired location for monitoring and assisting the said persons, this unit being equipped with a receiver and being intended to transmit an alarm signal, in particular by way of the switched telephone network (16), to one or more defined locations (17), in particular a monitoring centre, an emergency centre, neighbours or family;
- and a portable unit (1) intended to be carried by the said persons and incorporating an emitter which is intended to emit one or more signals in the direction of the decentralized unit (15), the said signals being intended to initiate the transmission of the alarm signal by way of the said decentralized unit in the direction of the said defined locations (17),
**characterized in that** the portable unit (1) additionally incorporates means (2, 3, 4) for detecting that the person carrying the device has fallen, these means being able to initiate the emission, by the emitter incorporated therein, of the said signals which are intended in turn to initiate the transmission of an alarm by the decentralized unit (15), these means of detection consisting of accelerometric means consisting of one or more piezoelectric accelerometers (2).

2. System for monitoring and assisting isolated persons according to Claim 1, **characterized in that** the portable unit includes three accelerometers (2), situated in three different directions, in such a way as to detect falls in the vertical plane, falls in the lateral plane and backward falls.

3. System for monitoring and assisting isolated persons according to one of Claims 1 and 2, **characterized in that** the portable unit (1) is also equipped with a member (13) which can be activated by the person carrying it and which is intended to initiate the transmission of an alarm by the decentralized unit (15).

4. System for monitoring and assisting isolated persons according to Claim 3, **characterized in that** the activatable member (13) consists of a press-button.

5. System for monitoring and assisting isolated persons according to Claim 3, **characterized in that** the portable unit is hung from a neck chain, and **in that** the activatable member (13) is incorporated in the device for closing the said chain, so that pulling the said chain off will trigger the alarm signal.

6. System for monitoring and assisting isolated persons according to one of Claims 1 to 5, **characterized in that** the portable unit is fixed by adhesive on the skin of the person to be monitored and assisted, in the fashion of a patch.

7. System for monitoring and assisting isolated persons according to one of Claims 1 to 6, **characterized in that** the portable unit includes an electrical alert circuit which is closed by a cutaneous impedance switch, so that the said portable unit is in the alert state only when it is being worn.

8. System for monitoring and assisting isolated persons according to one of Claims 1 to 7, **characterized in that** the portable unit (1) incorporates a circuit for recovery (10, 11) and storage (8, 9) of the electrocardiogram of the person in question.

9. System for monitoring and assisting isolated persons according to Claim 8, **characterized in that** the portable unit (1) is provided with a chain which is placed around the neck of the person in question, the said chain being equipped at its end, that is to say at the end opposite the area of attachment of the portable unit, and thus, when it is in place behind the neck, with contact electrodes which are able to take a first information item within the context of the recovery of the electrocardiogram, and **in that** the casing constituting the portable unit itself includes an electrode, situated on its rear face, so as to be situated level with the manubrium of the sternum of the individual, so as to permit, in association with the electrode for the neck, the acquisition of the data necessary for creating an electrocardiogram, the latter being stored in an integrated memory within the said casing.

10. System for monitoring and assisting isolated persons according to Claim 9, **characterized in that** the portable unit includes a logic and control section which is able, in particular, to analyse the acquired electrocardiogram automatically and in real time, in order to detect cardiac dysfunction and thus to initiate the triggering of an alarm signal.

11. System for monitoring and assisting isolated persons according to one of Claims 1 to 10, **characterized in that** the decentralized unit is mounted on the wheelchair of a handicapped person, the interface with the switched network being of the type used for operating mobile phones.

12. System for monitoring and assisting isolated persons according to one of Claims 1 to 11, **characterized in that** it includes a device for position-finding by satellite.

13. System for monitoring and assisting isolated persons according to one of Claims 1 to 12, **characterized in that** it includes means (12) making it possible to inform the said person or persons that the alarm signal has indeed been transmitted to the monitoring centre, emergency centre or to the designated third parties.

14. System for monitoring and assisting isolated persons consisting of a portable unit (1) incorporating an emitter which is intended to emit one or more signals capable of initiating the transmission of an alarm signal in the direction of a monitoring centre, emergency centre or third party, **characterized in that** it additionally incorporates means (2, 3, 4) for detecting that the person carrying the device has fallen, these means being able to initiate the emission, by the emitter incorporated therein, of the said signals which are intended to initiate the transmission of an alarm, these means of detection consisting of accelerometric means. consisting of one or more piezoelectric accelerometers (2).

15. System for monitoring and assisting isolated persons according to Claim 14, **characterized in that** it is equipped with a chain which is placed around the neck of the person in question, the said chain being equipped at its end, that is to say at the end opposite the area of attachment of the portable unit, and thus, when it is in place behind the neck, with contact electrodes which are able to take a first information item within the context of the recovery of the electrocardiogram, and **in that** it itself includes an electrode, situated on its rear face, so as to be situated level with the manubrium of the sternum of the individual, so as to permit, in association with the electrode for the neck, the acquisition of the data necessary for creating an electrocardiogram, the latter being stored in an integrated memory within the said casing.

16. System for monitoring and assisting isolated persons according to one of Claims 14 and 15, **characterized in that** the portable unit includes an electrical alert circuit which is closed by a cutaneous impedance switch, so that the said portable unit is in the alert state only when it is being worn.

## Patentansprüche

1. System zur Überwachung und Unterstützung isolierter Personen mit:
- einer im Bereich des gewünschten Überwachungs- oder Unterstützungsortes für die genannten Personen ortsungebundenen Einheit (15), die mit einem Empfänger ausgerüstet und dazu bestimmt ist, ein Alarmsignal an einen oder mehrere bestimmte Orte (17), insbesondere Überwachungszentren, Notdienste, Nachbarn, Familienangehörige, zu übertragen, insbesondere mittels des Fernsprechwählnetzes (16),
- und einer tragbaren Einheit (1), die dazu bestimmt ist, von den genannten Personen getragen zu werden, und einen Sender umfaßt, der dazu bestimmt ist, ein oder mehrere Signale in Richtung der ortsunabhängigen Einheit (15) zu senden, wobei die genannten Signale dazu bestimmt sind, die Übertragung des Alarmsignals mittels der genannten ortsunabhängigen Einheit in Richtung der genannten bestimmten Orte (17) auszulösen;
**dadurch gekennzeichnet, daß** die tragbare Einheit (1) weiterhin Mittel (2,3,4) zur Erfassung des Sturzes der Person, die sie trägt, umfaßt, welche dazu geeignet sind, das Versenden der genannten Signale durch den integrierten Sender auszulösen, welche Signale ihrerseits dazu bestimmt sind, die Übertragung des Alarms durch die ortsunabhängige Einheit (15) auszulösen, wobei diese Erfassungsmittel von Beschleunigungsmeßmitteln, bestehend aus einem oder mehreren piezoelektrischen Beschleunigungsmessern (2), gebildet werden.

2. System zur Überwachung und Unterstützung isolierter Personen nach Anspruch 1, **dadurch gekennzeichnet, daß** die tragbare Einheit drei Beschleunigungsmesser (2) aufweist, die entlang dreier unterschiedlicher Richtungen angeordnet sind, so daß Stürze in der vertikalen Ebene, Stürze in der seitlichen Ebene und Stürze von vorne nach hinten erfaßt werden.

3. System zur Überwachung und Unterstützung isolierter Personen nach einem der Ansprüche 1 bis 2, **dadurch gekennzeichnet, daß** die tragbare Einheit (1) ebenfalls mit einer durch die sie tragende Person betätigbaren Einrichtung (13) versehen ist, welche dazu bestimmt ist, die Übertragung des Alarms durch die ortsunabhängige Einheit (15) auszulösen.

4. System zur Überwachung und Unterstützung isolierter Personen nach Anspruch 3, **dadurch gekennzeichnet, daß** die betätigbare Einrichtung (13) von einem Druckknopf gebildet wird.

5. System zur Überwachung und Unterstützung isolierter Personen nach Anspruch 3, **dadurch gekennzeichnet, daß** die tragbare Einheit an einem Halsband oder einer Halskette aufgehängt ist und daß die betätigbare Einrichtung (13) in die Verschlußvorrichtung für das genannte Halsband oder die genannte Halskette integriert ist, wobei das Abreißen des genannten Halsbandes oder der genannten Halskette die Erzeugung des Alarmsignals zur Folge hat.

6. System zur Überwachung und Unterstützung isolierter Personen nach einem der Ansprüche 1 bis 5, **dadurch gekennzeichnet, daß** die tragbare Einheit durch einen Klebstoff nach Art eines Klebe-Patches auf der Haut der zu überwachenden und unterstützenden Person befestigt ist.

7. System zur Überwachung und Unterstützung isolierter Personen nach einem der Ansprüche 1 bis 6, **dadurch gekennzeichnet, daß** die tragbare Einheit einen elektrischen Überwachungsschaltkreis aufweist, der durch einen über die Hautimpedanz wirkenden Unterbrecher geschlossen ist, so daß die genannte tragbare Einheit sich nur im Überwachungszustand befindet, während sie getragen wird.

8. System zur Überwachung und Unterstützung isolierter Personen nach einem der Ansprüche 1 bis 7, **dadurch gekennzeichnet, daß** die tragbare Einheit (1) einen Schaltkreis zur Aufnahme (10,11) und Speicherung (8,9) des Elektrokardiogramms der betroffenen Person umfaßt.

9. System zur Überwachung und Unterstützung isolierter Personen nach Anspruch 8, **dadurch gekennzeichnet, daß** die tragbare Einheit (1) mit einem Halsband oder einer Halskette versehen ist, welche sich um den Hals der betroffenen Person legt, wobei das genannte Halsband oder die genannte Halskette im Bereich seines Endes, d.h. im Bereich gegenüber der Zone zur Befestigung der tragbaren Einheit und somit, wenn sie hinter dem Hals angeordnet ist, mit Kontaktelektroden versehen ist, welche geeignet sind, eine erste Information im Rahmen der Aufnahme des Elektrokardiogramms zu erfassen, und daß das die tragbare Einheit bildende Gehäuse selbst eine Elektrode umfaßt, welche auf ihrer dorsalen Fläche angeordnet ist, um im Bereich des Manubrium sterni des Individuums angeordnet zu sein, so daß er in Verbindung mit der Halselektrode die Aufnahme der zur Erstellung eines Elektrokardiogramms, welches in einem innerhalb des Gehäuses angeordneten Speicher abgespeichert wird, erforderlichen Daten erlaubt.

10. System zur Überwachung und Unterstützung isolierter Personen nach Anspruch 9, **dadurch gekennzeichnet, daß** die tragbare Einheit einen Logikbaustein zur Steuerung aufweist, der insbesondere geeignet ist, automatisch und in Echtzeit das aufgenommene Elektrokardiogramm zu analysieren, um Herzfehlfunktionen nachzuweisen und folglich die Erzeugung eines Alarmsignals auszulösen.

11. System zur Überwachung und Unterstützung isolierter Personen nach einem der Ansprüche 1 bis 10, **dadurch gekennzeichnet, daß** die ortsunabhängige Einheit im Bereich des Rollstuhls einer behinderten Person angebracht ist, wobei die Schnittstelle mit dem Wählnetz derart ist, wie sie für die Funktion von Mobiltelefonen eingesetzt werden.

12. System zur Überwachung und Unterstützung isolierter Personen nach einem der Ansprüche 1 bis 11, **dadurch gekennzeichnet, daß** es eine Satellitenortungsvorrichtung umfaßt.

13. System zur Überwachung und Unterstützung isolierter Personen nach einem der Ansprüche 1 bis 12, **dadurch gekennzeichnet, daß** es Mittel (12) aufweist, welche es erlauben, die genannte Person oder genannten Personen zu informieren, daß das Alarmsignal erfolgreich an das Überwachungszentrum, den Notdienst oder an bestimmte Dritte übertragen wurde.

14. System zur Überwachung und Unterstützung isolierter Personen, bestehend aus einer tragbaren Einheit (1), welche einen Sender enthält, der zum Senden eines oder mehrerer Signale bestimmt ist, die zur Auslösung der Übertragung eines Alarmsignals in Richtung eines Überwachungszentrums, eines Notdienstes oder an Dritte geeignet sind, **dadurch gekennzeichnet, daß** es ferner Mittel zur Erfassung (2,3,4) des Sturzes der die Einheit tragenden Person enthält, welche geeignet sind, durch den in ihm enthaltenen Sender die Aussendung der genannten Signale auszulösen, welche dazu bestimmt sind, die Übertragung des Alarms auszulösen, wobei diese Erfassungsmittel aus Beschleunigungsmeßmitteln besteht, welche einen oder mehrere piezoelektrische Beschleunigungsmesser (2) aufweisen.

15. System zur Überwachung und Unterstützung isolierter Personen nach Anspruch 14, **dadurch gekennzeichnet, daß** es mit einem Halsband oder einer Halskette versehen ist, welche sich um den Hals der betroffenen Person legt, wobei das genannte Halsband oder die genannte Halskette im Bereich ihres Endes, d.h. in dem der Befestigungszone der tragbaren Einheit gegenüberliegenden Bereich und somit, wenn sie hinter dem Hals angeordnet ist, Kontaktelektroden aufweist, die dazu geeignet sind, eine erste Information im Rahmen der Erfassung des Elektrokardiogramms aufzunehmen, und daß es selbst eine Elektrode aufweist, die auf seiner dorsalen Fläche angeordnet ist, um im Bereich des Manubrium sterni des Individuums zu liegen, um in Verbindung mit der Halselektrode die Aufnahme der zur Herstellung eines Elektrokardiogramms, welches in einem in das Gehäuse integrierten Speicher abgespeichert wird, erforderlichen Daten zu ermöglichen.

16. System zur Überwachung und Unterstützung isolierter Personen nach einem der Ansprüche 14 und 15, **dadurch gekennzeichnet, daß** die tragbare Einheit einen elektrischen Überwachungsschaltkreis aufweist, der durch einen über die Hautimpedanz wirkenden Unterbrecher geschlossen ist, so daß die genannte tragbare Einheit sich nur im Überwachungszustand befindet, während sie getragen wird.
